# EUROPEAN PATENT APPLICATION

(11) **EP 4 814 980 A2**
(43) Date of publication of application: **30.09.2026**
(21) Application number: 26189018.0
(22) Date of filing: 25.05.2023
(51) Int. Cl.: G16H 40/63

(54) **ASSESSMENT OF PAST INSULIN DELIVERY OUTCOMES TO AUTOMATICALLY TUNE MDA SYSTEMS**

(30) Priority: 27.05.2022 US 202263346363 P
(62) Document number(s) of the earlier application(s) in accordance with Art. 76 EPC: 23175229.6 / 4 283 624
(71) Applicant: Insulet Corporation, Acton, MA 01720 (US)
(72) Inventor: LEE, Joon Bok, Acton (US); O'CONNOR, Jason, Acton (US); ZHENG, Yibin, Hartland (US); ZADE, Ashutosh, San Diego (US)
(74) Representative: Peterreins Schley

(57) **Abstract**

Disclosed are techniques, devices and systems that obtain a glucose measurement history and a liquid drug delivery history. An expected drug delivery amount may be calculated based on the obtained glucose measurement history and the obtained liquid drug delivery history. A processor may calculate a plurality of respective drug delivery amounts implemented using different advisory mode algorithms. A respective advisory drug delivery amount of the plurality of respective advisory drug delivery amounts may be selected by the processor. A recommendation may be generated based on the selected respective advisory drug delivery amount.

## Description

### BACKGROUND

Assessment of the performance of a medication delivery algorithm (MDA), such as automatic insulin delivery (AID) algorithms, are often executed in a stepwise process, where a significant amount of insulin delivery data and blood glucose measurement data is processed. Changes in the AID algorithm that can result in improved glucose control outcomes are applied. However, the processing of significant amount of data may result in a significant delay in the time that it takes for each AID algorithm to improve its behaviors.

It would be beneficial if there was a process by which the behavior of an AID algorithm could be assessed in real time based on near term retrospective insulin delivery history and glucose control outcomes.

### BRIEF SUMMARY

This Summary is provided to introduce a selection of concepts in a simplified form that are further described below in the Detailed Description. This Summary is not intended to identify key features or essential features of the claimed subject matter, nor is it intended as an aid in determining the scope of the claimed subject matter.

A controller of a drug delivery system is disclosed. The controller includes a processor operable to execute programming instructions, a user interface including a display, a memory operable to store the programming instructions; and a communication circuitry coupled to the processor operable to receive and transmit wireless communication signals. The processor is operable to obtain historical data related to blood glucose measurement values and insulin delivery information. An expected insulin delivery amount for a current operational cycle is calculated using the historical data related to the blood glucose measurement values and insulin delivery information. A number of advisory mode algorithms are executed, and each advisory mode algorithm of the number of advisory mode algorithms utilizes different parameters to generate a respective advisory insulin delivery output. The respective advisory insulin delivery output from each advisory mode algorithm of the plurality of advisory mode algorithms is evaluated with respect to the expected insulin delivery. Based on a result of the evaluating, one respective advisory insulin delivery output is selected from the plurality advisory mode algorithms. Settings of a medication delivery algorithm are updated with parameters from an advisory mode algorithm that generated the selected one respective advisory insulin delivery output.

According to an example of the disclosed subject matter, a non-transitory computer readable medium is disclosed that embodies programming instructions executable by a processor that cause the processor to obtain a glucose measurement history and a liquid drug delivery history. An expected drug delivery amount may be calculated based on the obtained glucose measurement history and the obtained liquid drug delivery history. The processor is caused to calculate a plurality of respective drug delivery amounts implemented using different advisory mode algorithms. A respective advisory drug delivery amount of the plurality of respective advisory drug delivery amounts may be selected by the processor. A recommendation may be generated based on the selected respective advisory drug delivery amount.

A method is disclosed that includes calculating, by a processor, in particular by a processor of a drug delivery system or by a processor of a drug delivery device, an amount of a liquid drug that should have been delivered by the drug delivery device during a previous delivery cycle, based on subsequent blood glucose data, and executing a plurality of advisory algorithms. Each respective advisory algorithm of the plurality of advisory algorithms has different parameter settings and is operable to generate an estimated liquid drug amount expected to have been delivered during the previous delivery cycle. The estimated liquid drug amount generated by each respective advisory algorithm is compared to the determined amount of the liquid drug expected to have been delivered. The respective advisory algorithm is selected as a result of the comparison indicates generated based on the estimated liquid drug amount that most closely matched the determined amount of the liquid drug that should have been delivered during the previous delivery cycle. Parameter settings of the selected respective advisory algorithm are used in the calculation of a next amount of the liquid drug to be delivered by the drug delivery device during a next delivery cycle.

### BRIEF DESCRIPTION OF THE DRAWINGS

FIG. 1 illustrates an example of a system that is suitable to implement the subject matter described herein.
FIG. 2 illustrates an example of an adaptivity adjustment process according to the disclosed subject matter.

### DETAILED DESCRIPTION

The following discussion provides a framework to assess the behavior of an AID algorithm in real time based on near-term retrospective insulin delivery history and glucose control outcomes through advisory mode assessments and describes the incorporation of changes to the AID algorithm that retrospectively shows improved performance in historical data, which also holds promise in improving future delivery data as well.

A type of medication delivery algorithm (MDA) may include an "artificial pancreas" algorithm-based system, or more generally, an artificial pancreas (AP) application. For ease of discussion, the computer programs and computer applications that implement the medication delivery algorithms or applications may be referred to herein as an "AP application." An AP application may be configured to provide automatic delivery of insulin based on an analyte sensor input, such as signals received from an analyte sensor, such as a continuous blood glucose monitor, or the like. The signals from the analyte sensor may contain blood glucose measurement values, timestamps, or the like.

In addition, or alternatively, while the disclosed examples may have been described with reference to a closed loop algorithmic implementation, variations of the disclosed examples may be implemented to enable open loop use. The open loop implementations allow for use of different modalities of delivery of insulin such as smart pen, syringe or the like. For example, the disclosed AP application and algorithms may be operable to perform various functions related to open loop operations, such as the generation of prompts requesting the input of information such as diabetes type, weight or age. Similarly, a dosage amount of insulin may be received by the AP application or algorithm from a user via a user interface. Other open-loop actions may also be implemented by adjusting user settings or the like in an AP application or algorithm.

Systems, devices, computer readable media and methods in accordance with the present disclosure are now described more fully hereinafter with reference to the accompanying drawings, where one or more examples are shown. The systems, devices, and methods described herein may be embodied in many different forms and are not to be construed as being limited to the examples set forth herein. Instead, these examples are provided so the disclosure is thorough and complete, and may fully convey the scope of the techniques and devices to those skilled in the art. Each of the systems, devices, media and methods disclosed herein provides one or more advantages over conventional systems, components, and methods.

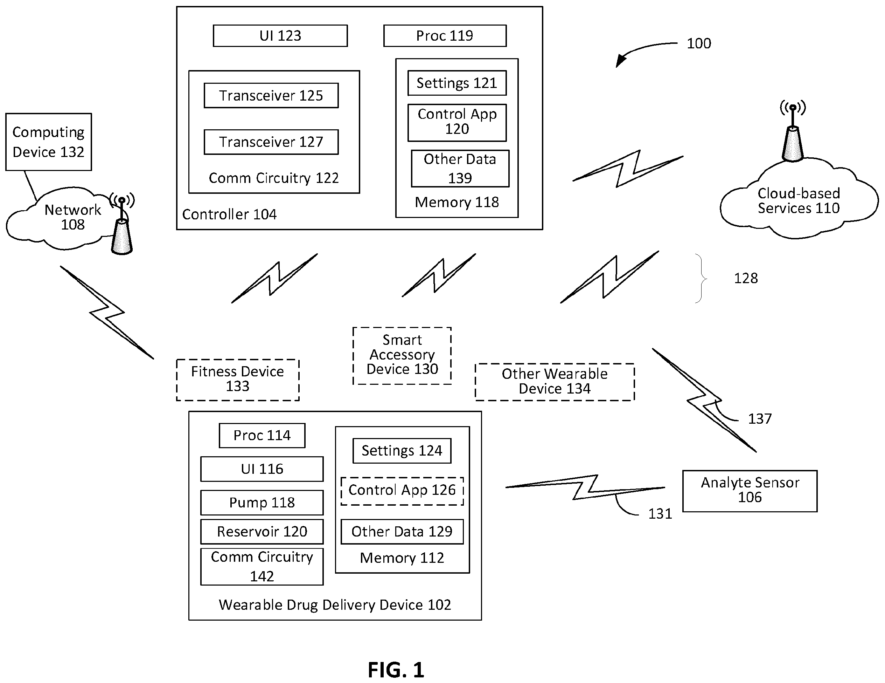

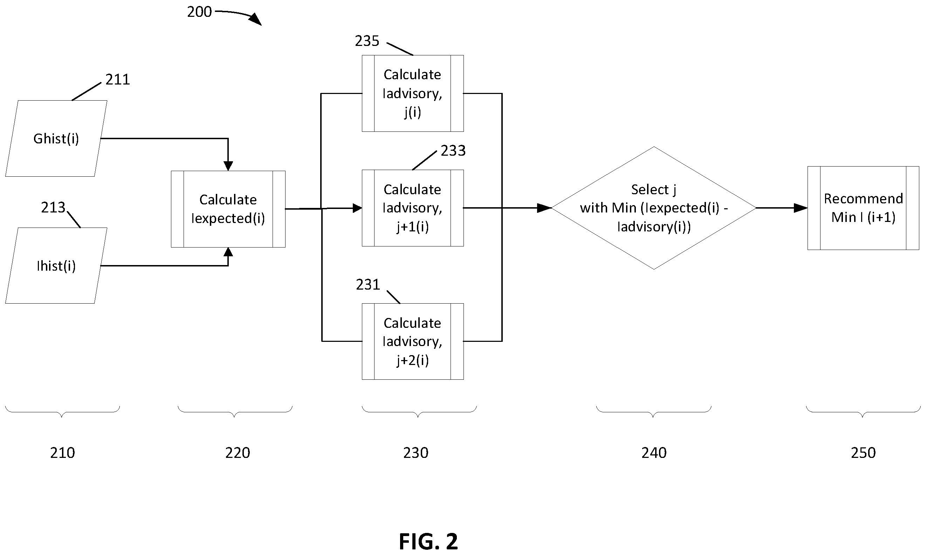

FIG. 1 illustrates a drug delivery system operable to implement the examples of FIGs. 1 and 2.

In some examples, the drug delivery system 100 is suitable for delivering insulin to a user in accordance with the disclosed embodiments. The drug delivery system 100 may include a wearable drug delivery device 102, a controller 104 and an analyte sensor 106.

The wearable drug delivery device 102 may be a wearable device that is worn on the body of the user. The wearable drug delivery device 102 may be directly coupled to a user (e.g., directly attached to a body part and/or skin of the user via an adhesive, or the like). In an example, a surface of the wearable drug delivery device 102 may include an adhesive to facilitate attachment to the skin of a user.

The wearable drug delivery device 102 may include a processor 114. The processor 114 may be implemented in hardware, software, or any combination thereof. The processor 114 may, for example, be a microprocessor, a logic circuit, a field programmable gate array (FPGA), an application specific integrated circuit (ASIC) or a microprocessor coupled to a memory. The processor 114 may maintain a date and time as well as be operable to perform other functions (e.g., calculations or the like). The processor 114 may be operable to execute a control application 126 stored in the memory 112 that enables the processor 114 to direct operation of the wearable drug delivery device 102. The control application 126 may control insulin delivery to the user per an AID control approach as describe herein. For example, the control application 126 may be an AID algorithm. The memory 112 may hold settings 124 for a user, such as specific factor settings, subjective insulin need parameter settings, AID algorithm settings, such as maximum insulin delivery, insulin sensitivity settings, total daily insulin (TDI) settings and the like. The memory may also store other data 129, such as total daily insulin values, blood glucose measurement values from analyte sensor 106 or controller 104, insulin dosage amounts (both basal and bolus) and the like from previous minutes, hours, days, weeks, or months. The analyte sensor 106 may be operable to collect physiological condition data, such as the blood glucose measurement values and a timestamp, that may be shared with the wearable drug delivery device 102, the controller 104 or both. For example, the communication circuitry 142 of the wearable drug delivery device 102 may be operable to communicate with the analyte sensor 106 and the controller 104 as well as the devices 130, 133 and 134. The communication circuitry 142 may be operable to communicate via Bluetooth, cellular communication, and/or other wireless protocols. While not shown, the memory 112 may include both primary memory and secondary memory. The memory 112 may include random access memory (RAM), read only memory (ROM), optical storage, magnetic storage, removable storage media, solid state storage or the like.

The wearable drug delivery device 102 may include a reservoir 120. The reservoir 120 may be operable to store drugs, medications or therapeutic agents suitable for automated delivery, such as insulin, morphine, methadone, hormones, glucagon, glucagon-like peptide, blood pressure medicines, chemotherapy drugs, arthritis drugs, combinations of drugs, such as insulin and glucagon-like peptide, or the like. A fluid path to the user may be provided via tubing and a needle/cannula (not shown). The fluid path may, for example, include tubing coupling the wearable drug delivery device 102 to the user (e.g., via tubing coupling a needle or cannula to the reservoir 120). The wearable drug delivery device 102 may be operable based on control signals from the processor 114 to expel the drugs, medications or therapeutic agents, such as insulin, from the reservoir 120 to deliver doses of the drugs, medications or therapeutic agents, such as the insulin, to the user via the fluid path. The processor 114 may be operable to cause insulin to be expelled from the reservoir 120.

There may be one or more communications links 128 with one or more devices physically separated from the wearable drug delivery device 102 including, for example, a controller 104 of the user and/or a caregiver of the user and/or a sensor 106. The communication links 128 may include any wired or wireless communication link operating according to any known communications protocol or standard, such as Bluetooth^{®}, Wi-Fi, a near-field communication standard, a cellular standard, or any other wireless protocol. The analyte sensor 106 may communicate with the wearable drug delivery device 102 via a wireless communication link 131 and/or may communicate with the controller 104 via a wireless communication link 137.

The wearable drug delivery device 102 may also include a user interface 116, such as an integrated display device for displaying information to the user, and in some embodiments, receiving information from the user. For example, the user interface 116 may include a touchscreen and/or one or more input devices, such as buttons, knobs or a keyboard that enable a user to provide an input.

In addition, the processor 114 may be operable to receive data or information from the analyte sensor 106 as well as other devices that may be operable to communicate with the wearable drug delivery device 102. The processor 114 may be operable to perform the functions, calculations and communications described later with reference to the examples of FIG. 2. For example, the memory 112 may store as settings 124 and/or other data 129 the information collected and calculated as described in the examples of FIG. 2. The control application 126 hosted in the wearable drug delivery device 102 may have programming code or may have access to programming code (e.g., via cloud-based services 110) that is executable by the processor 114 and enables execution of the functions described with reference to FIG. 2. In a further example, the programming instructions may be received via communication circuitry 142 and stored in memory 112 for execution by the processor 114.

The wearable drug delivery device 102 may interface with a network 108. The network 108 may include a local area network (LAN), a wide area network (WAN) or a combination therein. A computing device 132 may be interfaced with the network, and the computing device may communicate with the insulin delivery device 102. The computing device 132 may be a healthcare provider device through which a user's controller 104 may interact to obtain information, store settings and the like. The AID algorithm operating, as or in cooperation with, the control application 120 may present a graphical user interface on the computing device 132 enabling the input and presentation of information related to the AID algorithm.

The drug delivery system 100 may include an analyte sensor 106 for sensing the levels of one or more analytes of a user. The analyte levels may be used as physiological condition data and be sent to the controller 104 and/or the wearable drug delivery device 102. The sensor 106 may be coupled to the user by, for example, adhesive or the like and may provide information or data on one or more medical conditions and/or physical attributes of the user. The sensor 106 may be a continuous glucose monitor (CGM), or another type of device or sensor that provides blood glucose measurements that is operable to provide blood glucose concentration measurements. The sensor 106 may be physically separate from the wearable drug delivery device 102 or may be an integrated component thereof. The sensor 106 may provide the processor 114 and/or processor 119 with physiological condition data indicative of measured or detected blood glucose levels of the user. The information or data provided by the sensor 106 may be used to modify an insulin delivery schedule and thereby cause the adjustment of drug delivery operations of the wearable drug delivery device 102.

The drug delivery system 100 may also include the controller 104. In the depicted example, the controller 104 may include a processor 119 and a memory 118. The controller 104 may be a special purpose device, such as a dedicated personal diabetes manager (PDM) device. The controller 104 may be a programmed general-purpose device that is a portable electronic device, such as any portable electronic device, smartphone, smartwatch, fitness device, tablet or the like including, for example, a dedicated processor, such as processor, a micro-processor or the like. The controller 104 may be used to program or adjust operation of the wearable drug delivery device 102 and/or the sensor 106. The processor 119 may execute processes to manage a user's blood glucose levels and for control the delivery of the drug or therapeutic agent to the user. The processor 119 may also be operable to execute programming code stored in the memory 118. For example, the memory 118 may be operable to store a control application 120, such as an AID algorithm for execution by the processor 119. The control application 120 may be responsible for controlling the wearable drug delivery device 102, including the automatic delivery of insulin based on recommendations and instructions from the AID algorithm, such as those recommendations and instructions described herein.

The memory 118 may store one or more applications, such as control application 120, and settings 121 for the insulin delivery device 102 like those described above. In addition, the memory 118 may be operable to store other data and/or computer programs 139, such as drug delivery history, blood glucose measurement values over a period of time, total daily insulin values, and the like. For example, the memory 118 is coupled to the processor 119 and operable to store programming instructions, such as the control application 120 and settings 121, and data, such as other data 139, related to a blood glucose of a user and/or data related to an amount of insulin expelled by the wearable drug delivery device 102.

The controller 104 may include a user interface (UI) 123 for communicating with the user. The user interface 123 may include a display, such as a touchscreen, for displaying information. The touchscreen may also be used to receive input when it is a touch screen. The user interface 123 may also include input elements, such as a keyboard, button, knob or the like. In an operational example, the user interface 123 may include a touchscreen display controllable by the processor 119 and be operable to present the graphical user interface, and in response to the received input, the touchscreen display is operable to generate a signal indicative of the subjective insulin need parameter. The touchscreen display, under control of the processor 119, may be operable to, in response to the received input, generate a signal indicative of the subjective insulin need parameter as described with reference to earlier examples.

The controller 104 may interface via a wireless communication link of the wireless communication links 128 with a network, such as a LAN or WAN or combination of such networks that provides one or more servers or cloud-based services 110 via communication circuitry 122. The communication circuitry 122, which may include transceivers 127 and 125, may be coupled to the processor 119. The communication circuitry 122 may be operable to transmit communication signals (e.g., command and control signals) to and receive communication signals (e.g., via transceivers 127 or 125) from the wearable drug delivery device 102 and the analyte sensor 106. In an example, the communication circuitry 122 may include a first transceiver, such as 125, that may be a Bluetooth transceiver, which is operable to communicate with the communication circuitry 122 of the wearable drug delivery device 102, and a second transceiver, such as 127, that may be a cellular or Wi-Fi transceiver operable to communicate via the network 108 with computing device 132 or with cloud-based services 110.

The cloud-based services 110 may be operable to store user history information, such as blood glucose measurement values over a set period of time (e.g., days, months, years), a drug delivery history that includes insulin delivery amounts (both basal and bolus dosages) and insulin delivery times, types of insulin delivered, indicated meal times, blood glucose measurement value trends or excursions or other user-related diabetes treatment information, specific factor settings including default settings, present settings and past settings, or the like.

Other devices, like smart accessory device 130 (e.g., a smartwatch or the like), fitness device 133 and other wearable device 134 may be part of the drug delivery system 100. These devices may communicate with the wearable drug delivery device 102 to receive information and/or issue commands to the wearable drug delivery device 102. These devices 130, 133 and 134 may execute computer programming instructions to perform some of the control functions otherwise performed by processor 114 or processor 119. These devices 130, 133 and 134 may include user interfaces, such as touchscreen displays for displaying information such as current blood glucose level, insulin on board, insulin deliver history, or other parameters or treatment-related information and/or receiving inputs, such as those described with reference to the examples of FIGs. 1 and 2. The display may, for example, be operable to present a graphical user interface for providing input, such as request a change in basal insulin dosage or delivery of a bolus of insulin. These devices 130, 133 and 134 may also have wireless communication connections with the sensor 106 to directly receive blood glucose level data or receive in parallel a presentation of the graphical user interface as shown in FIG. 1.

In another operational example, the controller 104 may be operable to execute programming code that causes the processor 119 of the controller 104 to perform the following functions. The processor 119 of the controller 104 may execute an AID algorithm that is one of the control applications 120 stored in the memory or memory 118. The processor may be operable to present, on a user interface that is at least one component of the user interface 123. The user interface 123 may be a touchscreen display controlled by the processor 119, and the user interface 123 is operable to present a graphical user interface that offers an input of a subjective insulin need parameter usable by the AID algorithm. The processor 119 may cause presentation on a user interface of a user device, a graphical user interface offering an input device that enables input of a subjective insulin need parameter. The AID algorithm may generate instructions for the pump 118 to deliver basal insulin to the user or the like.

The processor 119 is also operable to collect physiological condition data related to the user from sensors, such as the analyte sensor 106 or heart rate data, for example, from the fitness device 133 or the smart accessory device 130. In an example, the processor 119 executing the AID algorithm may determine a dosage of insulin to be delivered based on the collected physiological condition of the user and a specific factor determined based on the subjective insulin need parameter. The processor 119 may output a control signal via one of the transceivers 125 or 127 to the wearable drug delivery device 102. The outputted signal may cause the processor 114 to deliver command signals to the pump 118 to deliver an amount of related to the determined dosage of insulin in the reservoir 120 to the user based on an output of the AID algorithm. The processor 119 may also be operable to perform calculations regarding settings of the AID algorithm as discussed as herein. Modifications to the AID algorithm settings may be stored in the memory 118, for example, as settings 121.

A wearable drug delivery device 102, typically, has a lifecycle that is based on the amount of liquid drug that is stored in a reservoir 120 of the wearable drug delivery device 102 and/or the amount of the liquid drug delivered to the user. An AID application or algorithm may use a number of parameters, such as blood glucose measurement values, total daily insulin, insulin onboard, and the like, when making the determination of an amount of the liquid drug to have delivered. In an operational example, the processor 119 of the controller 104 may be operable to receive evaluate the effectiveness of the AID algorithm's control of the drug delivery device 102. For example, the processor 119 may be operable to utilize historical data accessible by the processor in an evaluation of past performance of the AID algorithm and generate recommendations for adjusting settings of the AID algorithm accordingly as described in more detail with reference to the example of FIG. 2).

While the system 100 is described with reference to delivery of insulin and the use of an AID algorithm, the system 100 may be operable to implement a drug delivery regimen via a medication delivery algorithm using a number of different liquid or therapeutic drugs. A liquid drug may be or include any drug in liquid form capable of being administered by a drug delivery device via a subcutaneous cannula, including, for example, insulin, glucagon-like peptide-1 (GLP-1), pramlintide, glucagon, co-formulations of two or more of glucagon, GLP-1, pramlintide, and insulin; as well as pain relief drugs, such as opioids or narcotics (e.g., morphine, or the like), methadone, blood pressure medicines, chemotherapy drugs, fertility drugs, or the like.

The discussion of FIG. 2 outlines a process that evaluates the performance of the AID algorithm based on historical information and recommends application of a version of an AID algorithm with.

At 210 of the flow diagram, a processor may obtain historical data related to blood glucose measurement values 211 and drug delivery information 213. The historical data related to the blood glucose measurement values 211 may, for example, include a sequence of blood glucose measurement values obtained at predetermined time intervals and a timestamp. The predetermined time intervals may, for example, be the cycle time during which a continuous blood glucose monitor may provide a blood glucose measurement value to the processor, or the like. For example, the timestamp may indicate a time when each blood glucose measurement value was obtained in the sequence of blood glucose measurement values. The AID algorithm may be operable to estimate future blood glucose measurement values based on a user's past blood glucose measurement values. In addition, a user's target setpoint for a blood glucose measurement value may also be pre-set based on predictions from a user's past setpoints and/or history. The historical data related to the insulin delivery information 213 includes a dosage of insulin that was delivered in correspondence with the obtaining of at least one blood glucose measurement value.

At 220, the processor may calculate an expected insulin delivery amount for a current operational cycle using the historical data related to the blood glucose measurement values and insulin delivery information. In an example, the current operational cycle may be a set time period during which a blood glucose measurement value is received and an instruction to deliver a dosage of insulin is output by the processor (as well as a drug delivery device delivering the dosage of insulin). When calculating an expected insulin delivery amount for the current operational cycle using the historical data related to the user's blood glucose measurement values and the user's insulin delivery information, the processor may, for example, be operable to determine a predicted future insulin delivery value that accounts for insulin onboard and peak insulin action.

In an example, the processor when calculating the expected insulin delivery may be operable to take the sum of all insulin deliveries made during a period of time prior to the current operational cycle. The period of time prior to the current operational cycle has a maximum threshold of a predetermined number of hours prior to the current operational cycle and a minimum threshold that is a set time prior to the current operational cycle. A user's additional insulin delivery needs may be added to the sum of all the insulin deliveries based on the user's current blood glucose measurement value (i.e., a most recently received blood glucose blood measurement value or the blood glucose measure value with a timestamp that is closest to the present time).

At 230, the processor may execute a number of advisory mode algorithms 231, 233 and 235. Each of the advisory mode algorithms 231, 233 and 235 may utilize different parameters (or input sets, such as an aggressive input set, a standard input set or a conservative input set for execution in a respective one of the number of advisory mode algorithms) to generate their respective advisory insulin delivery output. In certain implementations, a more aggressive input set may have a low glucose control target, whereas a more conservative input set may have a higher glucose control target. The low glucose control target may be the lowest investigated control target, such as 100 mg/dL, and the high glucose control target may be the highest investigated control target, such as 150 mg/dL. A respective advisory insulin delivery output may be an estimated amount of insulin that the respective advisory mode algorithm 231, 233 or 235 recommends being delivered to the user. In a specific example, advisory mode algorithm 231 may utilize parameters that may result in the algorithm generating an advisory insulin delivery output that may be considered "conservative" (e.g., *j* =1) in comparison to respective advisory insulin delivery outputs generated by other advisory mode algorithms. For example, the "conservative" advisory insulin delivery output may generate a recommendation to deliver an amount of insulin that is less than the amount of insulin delivered during the current operational cycle. Meanwhile, the advisory mode algorithm 233 may utilize parameters that generate a respective advisory insulin delivery output that is considered "standard" (e.g., *j* = 2) in comparison to "conservative" advisory mode algorithm 231. The "standard" advisory mode algorithm 233 may yield values substantially equal to the expected insulin delivery amount. The advisory algorithm 235 may utilize parameters that generate a respective advisory insulin delivery output that is considered "aggressive" (e.g., *j* =3). Of course, the number (i.e., *j*) of advisory mode algorithms may be a number other than 3, such as 2, 4, 5, 6, 10, 15 or the like.

Upon generation of the respective advisory insulin delivery output from each advisory mode algorithm of the plurality of advisory mode algorithms (e.g., *j* = 1 through 3), the processor may, for example, at 240, evaluate each respective advisory insulin delivery output with respect to the expected insulin delivery. In an example, the processor while evaluating the respective advisory insulin delivery output from each advisory mode algorithm of the plurality of advisory mode algorithms with respect to the expected insulin delivery, may be further operable to identify an estimated insulin amount that most closely matches the expected insulin delivery amount. For example, the processor may utilize a minimum function during the evaluation of the respective advisory insulin delivery outputs or the like. The minimum function may be configured to evaluate a difference between the expected insulin delivery and each respective advisory insulin delivery output. The processor, based on a result of the evaluating (e.g., the output of the minimum function), may select one respective advisory insulin delivery output from the plurality advisory mode algorithms. For example, in situations where the MDA algorithm over-delivered insulin (i.e., delivered too much) in the current operational cycle, the evaluation at 240 may indicate that the output from the "conservative" advisory mode algorithm 231 satisfies the minimum function and the processor may select the parameters of the "conservative" advisory mode algorithm 231 for inclusion in the MDA algorithm to determine a next operational cycle insulin delivery dosage amount.

At 250, the processor updates settings of the MDA algorithm with parameters from an advisory mode algorithm that generated the selected one respective advisory insulin delivery output. The medication delivery algorithm with the updated settings may be used to generate an insulin delivery dosage amount that is to be delivered during a next operational cycle after the current operational cycle. The next operational cycle may, for example, be the cycle that is immediately after the current operational cycle.

In a more detailed operational example, an MDA algorithm executed by a system such as 100 of Fig. 1, may have access to a history of the user's blood glucose measurements and a history of the insulin deliveries to the user (as illustrated at 210). In the example, an MDA algorithm executed by a processor may implement an equation that utilizes the user's blood glucose measurements and a history of the insulin deliveries. The equation may have a first term and a second term. The first term in the equation may be directed to the actual insulin deliveries by the drug delivery device over a period of time, such as a number of hours, and the second term in the equation may calculate an insulin deficit or excess insulin based on the history of the user's blood glucose measurements and insulin on board (IOB). In the example, these first and second terms may be individualized with respect to each user based on the user's various glucose-insulin dynamics, insulin sensitivities, and manual insulin delivery patterns. Based on the user respective insulin delivery history and the blood glucose measurement history, an assessment can be made by the processor, in real time or substantially real time, of whether the drug delivery system's overall insulin amount that was delivered in the last *h* hours (such as 5 hours) was appropriate for the user. Note that the assessment by the processor may, for example, utilize operational cycles of when a blood glucose measurement is received by the controller, in most systems, an operational cycle is approximately 5 minutes. Of course, operational cycles may be shorter, such as 1 minute, or longer, such as 10 minutes. The assessment may be implemented, as follows: ${I}_{expected} \left(i\right) = {\sum }_{k = 6}^{12 ⋅ h} I \left(i-k\right) + \frac{\left({\sum }_{k = 1}^{L} \frac{G \left(i+k\right) - SP \left(i+k\right)}{\frac{1800}{TDI} ⋅ L} - IOB \left(i\right)\right)}{12 ⋅ h / 18} ,$ (Equation 1), where *I_{expected}* is the insulin delivery expected for the current i^{th} cycle, G(i+k) is the AID system's prediction of future glucose values subsequent to the current cycle, the difference of G(i+k)-SP(i+k) is the deviation of the user's blood glucose measurements from the set point SP for the particular cycles being evaluated, the summation represents the total deviation over the cycles (i.e., *k=1-L*) making up the future period over which the deviations glucose predictions are being calculated (with a typical value of 12, or 1 hour, i.e., 12 5-minute cycles), insulin on board (IOB(i)) represents an amount of insulin during the particular cycle *i* that the user has yet to process, and the denominator value (i.e., 12*h/18) averages the deviation over a previous period of interest. Note that 18 represents 90 minutes (5 minutes *18), which is a common time period for insulin to take effect in the human body. In Equation 1, the summation of the past insulin deliveries represented by *I*(*i-k*) is shown to begin at *k*=6 to avoid counting the amount of insulin delivered in the most recent 30 minutes. Another period of time may be used.

In Equation 1, *I_{expected}* may represent what the user should have received in the last *h* hours to 30 minutes prior to the current time (that may correspond to the current operational cycle), with the understanding that insulin delivery in the last 30 minutes likely does not have a significant impact on the user's current glucose due to time delays within the biological system. The 30 minutes may be a tunable parameter and may be other values between 10 minutes to 60 minutes or the like. The expected insulin delivery (*I_{expected}*) is calculated by determining the sum of all insulin delivered over a period of insulin delivery time, such as between *h* hours ago to 30 minutes ago (i.e., I(i-k)), then adding the additional insulin delivery needs for the user based on the user's current glucose (i.e., the value to the right of the plus sign in Equation 1). The cycles under evaluation may be extended back into history; so, for example, instead of near-term retrospective analysis such as 5 hours, the analysis could go back days, weeks or even months (e.g., where h is 120 hours, 240 hours, 504 hours, 744 hours or the like).

The numerator ${\sum }_{k = 1}^{L} \frac{G \left(i+k\right) - SP \left(i+k\right)}{\frac{1800}{TDI} ⋅ L}$ of the second term in Equation 1 is a calculation of a user's estimated insulin need in the future. The tunable time range of *k* =1-Lis the number of operational cycles over which the user's insulin need is calculated. If L = 12, time range *k* =1-L, when an operational cycle is approximately equal to 5 minutes, is approximately 1 hour (12 x 5 minutes = 60 minutes). To account for the amount of insulin that has already been delivered or has not been delivered, the amount of insulin onboard (IOB) (i.e., the amount of insulin not yet processed by the body) for the current operational cycle (*i*) is subtracted from the user's predicted insulin need in the future as shown below: $PFI = \frac{\left({\sum }_{k = 1}^{L} \frac{G \left(i+k\right) - SP \left(i+k\right)}{\frac{1800}{TDI} ⋅ L} - IOB \left(i\right)\right)}{12 ⋅ h / 18}$ wherein the output is a predicted future insulin (PFI) delivery value that accounts for insulin onboard (IOB) for the current operational cycle (*IOB*(*i*)) and peak insulin action for the number of hours *h.* The variable *k* in the PFI calculation represents the *k=1 to L predicted* cycles of glucose deviation. The denominator (i.e., 12*·h* / 18) is a value that takes into account the peak insulin action of the delivered insulin. The value 12·*h* / 18 may scale in units of 90 minutes because it is anticipated that it takes approximately 90 minutes for insulin to act in the body (in terms of peak action).

To explain the PFI calculation in more detail, the user's current "ideal" additional insulin delivery needs are estimated by calculating the user's average predicted glucose deviations in the next *k* cycles and dividing it by the TDI-based correction factor, to calculate the estimated correction insulin needed for the user. This can be positive or negative. Existing IOB can be removed from this additional insulin need, as it is insulin that will act within the user later on. This additional insulin need is divided by the length of history that is being investigated, scaled by insulin peak time value, which in this example may be 90 minutes assuming insulin peak time.

The term "current" is often used with respect to a current insulin delivery need, or a current cycle or the like. As understood herein, "current" refers to the present time, a present value, such as a blood glucose measurement value or an estimated insulin dosage (basal or bolus), or a present setting, such as conservative or aggressive, or the like, and includes the understanding that the current time, current value and the current setting may change in the future. For example, a current operational cycle is the operational cycle that is presently being executed by the processor, which may include functions such as receiving blood glucose values, delivering a dose of basal insulin and/or a bolus insulin dosage, updating data stored in memory, and the like.

In more detail, the following logic, such as that to implement Equation 2 below, may be executed to assess various possible variations in the advisory mode algorithms discussed above. For example, advisory mode observations used in the advisory mode algorithms may incorporate prior clinical glucose traces (e.g., a number of past glucose values), giving point-by-point algorithm recommendations based on the input values to test for an automated insulin delivery system's prospective insulin delivery. Therefore, execution of the advisory mode allows the assessment on the impact of outputs if there are any changes to the interoperable automatic glucose control (iAGC) or input states under the same clinical conditions. As a result, advisory mode is a method that is widely used within the scientific field to evaluate the performance of AID systems that utilize an MDA algorithm. Specifically, as mentioned above, the advisory mode algorithms may formulate and execute a "conservative," a "standard," or an "aggressive" algorithm on existing data to compare the insulin delivery that would have been recommended by each algorithm versus the existing data. Or course, more granular algorithm formulations may also be set, such as subsets of respective algorithms (e.g., conservative 1 and conservative 1A, aggressive 2 and 3, or the like).

At all times, the insulin delivery recommendations of all the advisory algorithm settings (e.g., "conservative," "standard," or "aggressive") advisory algorithm settings (where *j* = 1-3) can be calculated as shown in Equation 2 below: ${I}_{advisory , j} \left(i\right) = {\sum }_{k = 6}^{12 ⋅ h} {I}_{manual} \left(i-k\right) + {I}_{alg , j} \left(i-k\right)$ where *Iₘₐₙᵤₐₗ* is a value of insulin delivered by manual command of the user (e.g., a user's meal boluses) in the past operational cycles (*i-k*) that were at least 30 minutes prior to the current operational cycle (i.e., *i*) and the amount of expected insulin (i.e., *I_{alg,j}*) to be delivered in the past except for the last 30 minutes prior to the current operational cycle (i.e., *i*). The "last 30 minutes" may be represented by the variable *k* starting at, for example, 6 and an operational cycle being, for example, equal to 5 minutes.

The insulin deviations between each MDA algorithm variant (*I_{alg,j}*(*i* - *k*)) and the expected ideal total insulin delivery may can be calculated. Finally, the output of the MDA algorithm variant with the minimum deviation between the sum of each algorithm's variations versus *I_{expected}* can be selected for insulin delivery recommendation in the current cycle.

In the cases where the user over-boluses (i.e., delivers too much insulin) and/or the system over-delivers basal, the processor may be operable to select, for example at 240 of FIG. 2 , the "conservative" advisory mode in an attempt to mitigate the user's over-bolusing and/or the system's basal over-delivery. Similarly, in instances, where the user under-boluses (i.e., delivers too little insulin), the "aggressive" advisory mode may be selected to attempt to mitigate the user's under-bolusing and/or the system's basal under-delivery. Meanwhile, if the evaluation at 220 of FIG. 4 indicates the MDA algorithm is performing close to the expected delivery values and the user is manually bolusing close to recommended or proper amounts, the output of the "standard" advisory mode may be selected by the processor.

In an overview, the MDA algorithm of a closed loop control system attempts to minimize the aggregate penalty of the cost function over a wide range of possible dosages. The cost function is applied to the possible dosages, and the dosage with the best cost function value is selected. Depending on how the cost function is configured, the best value may be the lowest value or the highest value. An example of a cost function used in determining an appropriate dosage is below.

The final glucose cost function for calculating the glucose cost component may be a weighted combination of a piecewise glucose cost function. The piecewise cost function is more heavily weighted when the expected insulin delivery is either closer to the "conservative" calculated advisory insulin delivery or to the "aggressive" calculated advisory insulin delivery. Thus, the weights dynamically adjust the balance between the piecewise cost function and the standard cost function based on advisory mode calculations.

The cost function may be adjusted to maintain the insulin to be delivered as close to the amount of insulin to be delivered as recommended by the advisory mode calculations. As a starting point for this discussion, it is helpful to review an exemplary cost function. An exemplary formulation for cost *J* is: $J = Q ⋅ {\sum }_{i = 1}^{M} G \left(i\right) + R ⋅ {\sum }_{i = 1}^{N} I \left(i\right)$ where *Q* is a weight coefficient of the glucose cost component (e.g., deviations of blood glucose from a set point value) and *R* is a weight coefficient of the insulin cost component (e.g., deviations of insulin amounts delivered from a standard basal amount), *G*(*i*) is the projected glucose level for an insulin dosage at cycle *i, M* is the number of cycles in the prediction horizon, *I*(*i*) is the projected insulin delivered at cycle *i,* and *N* is the control horizon in cycles. Thus, $Q ⋅ {\sum }_{i = 1}^{M} G \left(i\right)$ is the weighted glucose cost, and $R ⋅ {\sum }_{i = 1}^{n} I \left(i\right)$ is the weighted insulin cost. The prediction horizon *M* (i.e., the number of cycles in the future that the prediction is being made) and the control horizon *N* (i.e., the number of cycles in the future that the MDA algorithm will be controlling delivery) may be different numbers, may differ by one or more cycles, or may be equal. The total cost *J* is the sum of the weighted glucose cost and the weighted insulin cost. The total cost *J* is intended to be minimized by the algorithm for predicted glucose and insulin trends *G* and *I* over *M* and *N* cycles, respectively. As mentioned above, a cycle may have a fixed interval, such 5 minutes.

The weightings of the glucose cost and the insulin cost may be expressed as a ratio of Q:R. Different Q:R ratios can be incorporated as the "conservative," "standard" and "aggressive" variants:

| Algorithm Type | Q:R Ratio |
|---|---|
| Conservative | 18000 |
| Standard | 9000 |
| Aggressive | 4500 |

Based on the advisory algorithm, the respective Q:R ratios may be applied during steps 231, 233 and 235 and used in the calculation of a recommended insulin dosage.

Insulin may also be replaced by other drugs that are used for purposes other than the treatment of diabetes, such as chemotherapy drugs, pain relief drugs, such as opioids and narcotics, or other drugs referred to above. Of course, inputs to the above equations may be from sensors other than a continuous blood glucose monitor, such as a fingertip glucose sensor or a ketone sensor, but similar equations and actions may be taken.

Software related implementations of the techniques described herein, such as the processes examples described with reference to FIGs. 1 and 2 may include, but are not limited to, firmware, application specific software, or any other type of computer readable instructions that may be executed by one or more processors. The computer readable instructions may be provided via non-transitory computer-readable media. The various elements of the processes described with reference to the figures may be implemented in devices, apparatuses or systems that may include various hardware elements, software elements, or a combination of both. Examples of hardware elements may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, processes, procedures, software interfaces, application program interfaces (API), instruction sets, computing code, computer code, code segments, computer code segments, words, values, symbols, or any combination thereof.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), a tape, a cassette, or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

Although the present invention has been described above and is defined in the claims, it should be understood that the present invention can also be defined in accordance with the following embodiments:
1. A method, comprising:
   calculating, by a processor, in particular by a processor of a drug delivery system or by a processor of a drug delivery device, an amount of a liquid drug that should have been delivered by the drug delivery device during a previous delivery cycle;
   executing a plurality of advisory algorithms, wherein each respective advisory algorithm of the plurality of advisory algorithms has different parameter settings and is operable to generate an estimated liquid drug amount expected to have been delivered during the previous delivery cycle;
   comparing the estimated liquid drug amount generated by each respective advisory algorithm to the determined amount of the liquid drug expected to have been delivered;
   selecting the respective advisory algorithm that a result of the comparison indicates generated the estimated liquid drug amount that most closely matched the determined amount of the liquid drug that should have been delivered during the previous delivery cycle; and
   using parameter settings of the selected respective advisory algorithm in the calculation of a next amount of the liquid drug to be delivered by the drug delivery device during a next delivery cycle.
2. The method of embodiment 1, wherein calculating the amount of the liquid drug expected to have been delivered by the drug delivery device, comprises the steps of:
   calculating a user's average predicted glucose deviations in the next several cycles;
   dividing the user's average predicted glucose deviations in the next several cycles by a TDI-based correction factor, wherein the quotient is the estimated correction insulin needed for the user;
   determining a difference between an existing insulin on board from the estimated correction insulin needed for the user; and
   dividing the difference by a length of insulin delivery history being investigated, which is scaled by an insulin peak time value, wherein the quotient of the division is additional insulin delivery needs for the user.
3. The method of embodiment 1 or 2, wherein calculating the amount of the liquid drug expected to have been delivered by the drug delivery device, further comprises the steps of:
   determining a sum of all insulin delivered over a period of insulin delivery time; and
   adding the sum of all the insulin delivered over the period of insulin delivery time to the additional insulin delivery needs for the user, wherein the result of the adding is the amount of the liquid drug expected to have been delivered by the drug delivery device.
4. The method of one of embodiments 1 to 3, wherein a first respective advisory algorithm of the plurality of advisory algorithms includes aggressive parameter settings, a second respective advisory algorithm of the plurality of advisory algorithms includes standard parameter settings, and a third respective advisory algorithm of the plurality of advisory algorithms includes conservative parameter settings.
5. A non-transitory computer readable medium embodied with programming instructions executable by a processor that when executed by the processor cause the processor to:
   obtain a glucose measurement history;
   obtain a liquid drug delivery history;
   calculate an expected drug delivery amount based on the obtained glucose measurement history and the obtained liquid drug delivery history;
   calculate a plurality of respective drug delivery amounts implemented using different advisory mode algorithms;
   select a respective advisory drug delivery amount of the plurality of respective advisory drug delivery amounts; and
   generate a recommendation based on the selected respective advisory drug delivery amount.
6. The computer readable medium of embodiment 5, wherein the processor is further caused to:
   receive confirmation of the generated recommendation; and
   in response to the confirmation, output a control signal to deliver the selected respective advisory drug delivery amount.
7. The computer readable medium of embodiment 5 or 6, wherein the selecting the respective advisory drug delivery amount, the processor is further caused to:
   evaluate each respective advisory drug delivery amount of the plurality of respective advisory drug delivery amounts with respect the expected drug delivery amount; and,
   identify the respective advisory drug delivery amount of the plurality of respective advisory drug delivery amounts that most closely matches the expected drug delivery amount, wherein the identified respective advisory drug delivery amount is the selected respective advisory drug delivery amount.
8. The computer readable medium of one of embodiments 5 to 7, wherein the glucose measurement history includes glucose measurement data obtained over a plurality of measurement cycles.
9. The computer readable medium of one of embodiments 5 to 8, wherein the liquid drug delivery history includes liquid drug data based on deliveries of a liquid drug by a drug delivery device over a plurality of drug delivery cycles.
10. The computer readable medium of one of embodiments 5 to 9, wherein the programming instructs further cause the processor to:
   calculate each respective drug delivery amount using different sets of inputs in an advisory mode algorithm.
11. The computer readable medium of embodiments 5 to 10, wherein the different sets of inputs are categorized as an aggressive input set, a standard input set or a conservative input set for execution in the advisory mode algorithm.
12. A controller of a drug delivery system, comprising:
   a processor operable to execute programming instructions;
   a memory operable to store the programming instructions; and
   a communication circuitry coupled to the processor operable to receive and transmit wireless communication signals, wherein the processor when executing the programming instructions stored in the memory is operable to:
      obtain historical data related to blood glucose measurement values and insulin delivery information;
      calculate an expected insulin delivery amount for a current operational cycle using the historical data related to the blood glucose measurement values and insulin delivery information;
      execute a plurality of advisory mode algorithms, wherein each advisory mode algorithm of the plurality advisory mode algorithms utilizes different parameters to generate a respective advisory insulin delivery output;
      evaluate the respective advisory insulin delivery output from each advisory mode algorithm of the plurality of advisory mode algorithms with respect to the expected insulin delivery; and
      based on a result of the evaluating, select one respective advisory insulin delivery output from the plurality advisory mode algorithms; and
      update settings of a medication delivery algorithm with parameters from an advisory mode algorithm that generated the selected one respective advisory insulin delivery output.
13. The controller of the drug delivery system of embodiment 12, wherein the processor is further operable to:
   prior to updating the settings of the medication delivery algorithm, present the advisory mode algorithm that generated the selected one respective insulin delivery output as a recommended algorithm to generate an insulin delivery amount to be delivered in correspondence with a next operational cycle.
14. The controller of the drug delivery system of embodiment 12 or 13, wherein the historical data related to the blood glucose measurement values includes a sequence of blood glucose measurement values obtained at predetermined time intervals and a timestamp indicating a time when each blood glucose measurement value was obtained in the sequence of blood glucose measurement values.
15. The controller of the drug delivery system of one of embodiments 12 to 14, wherein the historical data related to the insulin delivery information includes a dosage of insulin that was delivered in correspondence with the obtaining of at least one blood glucose measurement value.
16. The controller of the drug delivery system of one of embodiments 12 to 15, wherein the current operational cycle is a set time period during which a blood glucose measurement value is received and an instruction to deliver a dosage of insulin is output by the processor.
17. The controller of the drug delivery system of one of embodiments 12 to 16, wherein the processor, when calculating an expected insulin delivery amount for the current operational cycle using the historical data related to the blood glucose measurement values and insulin delivery information, the processor is operable to:
   determine a predicted future insulin delivery value that accounts for insulin onboard and peak insulin action.
18. The controller of the drug delivery system of one of embodiments 12 to 17, wherein the processor, when evaluating the respective advisory insulin delivery output from each advisory mode algorithm of the plurality of advisory mode algorithms with respect to the expected insulin delivery, is further operable to:
   identify an estimated insulin amount that most closely matches the expected insulin delivery amount.
19. The controller of the drug delivery system of one of embodiments 12 to 18, wherein the processor, when calculating the expected insulin delivery amount for the current operational cycle using the historical data related to the blood glucose measurement values and insulin delivery information, is further operable to:
   taking the sum of all insulin deliveries made during a period of time prior to the current operational cycle, wherein the period of time prior to the current operational cycle has a maximum threshold of a predetermined number of hours prior to the current operational cycle and a minimum threshold that is a set time prior to the current operational cycle; and
   adding a user's additional insulin delivery needs to the sum of all the insulin deliveries based on the user's current blood glucose measurement value.
20. The controller of the drug delivery system of one of embodiments 12 to 19, wherein the processor, when calculating the expected insulin delivery amount for the current operational cycle using the historical data related to the blood glucose measurement values and insulin delivery information, is further operable to:
   summing a total amount of insulin delivered over a prior predetermined number of cycles to a current operational cycle;
   determine an average estimated future amount of insulin to be delivered over a number of future cycles, wherein the estimated future amount of insulin is offset by an amount of insulin onboard the user.
21. A non-transitory computer readable medium embodied with programming instructions executable by a processor that when executed by the processor cause the processor to:
   obtain a glucose measurement history;
   obtain a liquid drug delivery history;
   calculate an expected drug delivery amount based on the obtained glucose measurement history and the obtained liquid drug delivery history;
   calculate a plurality of respective drug delivery amounts implemented using different advisory mode algorithms;
   select a respective advisory drug delivery amount of the plurality of respective advisory drug delivery amounts; and
   generate a recommendation based on the selected respective advisory drug delivery amount.
22. The computer readable medium of embodiment 21, wherein the selecting the respective advisory drug delivery amount, the processor is further caused to:
   evaluate each respective advisory drug delivery amount of the plurality of respective advisory drug delivery amounts with respect the expected drug delivery amount; and,
   identify the respective advisory drug delivery amount of the plurality of respective advisory drug delivery amounts that most closely matches the expected drug delivery amount, wherein the identified respective advisory drug delivery amount is the selected respective advisory drug delivery amount.
23. A method, comprising:
   calculating, by a processor, in particular by a processor of a drug delivery system or by a processor of a drug delivery device, an amount of a liquid drug that should have been delivered by the drug delivery device during a previous delivery cycle;
   executing a plurality of advisory algorithms, wherein each respective advisory algorithm of the plurality of advisory algorithms has different parameter settings and is operable to generate an estimated liquid drug amount expected to have been delivered during the previous delivery cycle;
   comparing the estimated liquid drug amount generated by each respective advisory algorithm to the determined amount of the liquid drug expected to have been delivered;
   selecting the respective advisory algorithm that a result of the comparison indicates generated the estimated liquid drug amount that most closely matched the determined amount of the liquid drug that should have been delivered during the previous delivery cycle; and
   using parameter settings of the selected respective advisory algorithm in the calculation of a next amount of the liquid drug to be delivered by the drug delivery device during a next delivery cycle.
24. The method of embodiment 23, wherein calculating the amount of the liquid drug expected to have been delivered by the drug delivery device, comprises the steps of:
   calculating a user's average predicted glucose deviations in the next several cycles;
   dividing the user's average predicted glucose deviations in the next several cycles by a TDI-based correction factor, wherein the quotient is the estimated correction insulin needed for the user;
   determining a difference between an existing insulin on board from the estimated correction insulin needed for the user; and
   dividing the difference by a length of insulin delivery history being investigated, which is scaled by an insulin peak time value, wherein the quotient of the division is additional insulin delivery needs for the user.
25. The method of embodiment 23 or 24, wherein calculating the amount of the liquid drug expected to have been delivered by the drug delivery device, further comprises the steps of:
   determining a sum of all insulin delivered over a period of insulin delivery time; and
   adding the sum of all the insulin delivered over the period of insulin delivery time to the additional insulin delivery needs for the user, wherein the result of the adding is the amount of the liquid drug expected to have been delivered by the drug delivery device.
26. The method of one of embodiments 23 to 25, wherein a first respective advisory algorithm of the plurality of advisory algorithms includes aggressive parameter settings, a second respective advisory algorithm of the plurality of advisory algorithms includes standard parameter settings, and a third respective advisory algorithm of the plurality of advisory algorithms includes conservative parameter settings.

## Claims

1. A non-transitory computer readable medium embodied with programming instructions executable by a processor that when executed by the processor cause the processor to::
calculate, by a processor of a drug delivery device, an amount of a liquid drug that should have been delivered by the drug delivery device during a previous delivery cycle;
executing a plurality of advisory algorithms, wherein each respective advisory algorithm of the plurality of advisory algorithms has different parameter settings and is operable to generate an estimated liquid drug amount expected to have been delivered during the previous delivery cycle;
compare the estimated liquid drug amount generated by each respective advisory algorithm to the determined amount of the liquid drug that should have been delivered;
select the respective advisory algorithm that a result of the comparison indicates generated the estimated liquid drug amount that most closely matched the determined amount of the liquid drug that should have been delivered during the previous delivery cycle; and
use parameter settings of the selected respective advisory algorithm in the calculation of a next amount of the liquid drug to be delivered by the drug delivery device during a next delivery cycle.

2. The non-transitory computer readable medium of claim 1, wherein the expected liquid drug delivery amount is calculated by determining the sum of all liquid drug delivered over a period of delivery time and adding the additional liquid drug delivery needs for the user based on the user's current blood glucose measurement value.

3. The non-transitory computer readable medium of claim 2, wherein the sum of all liquid drug delivered is determined for a period of time prior to the current operational cycle, wherein the period of time prior to the current operational cycle has a maximum threshold of a predetermined number of hours prior to the current operational cycle and a minimum threshold that is a set time prior to the current operational cycle.

4. The non-transitory computer readable medium of any preceding claim, wherein the historical data related to blood glucose measurement values includes a sequence of blood glucose measurement values obtained at predetermined time intervals and a timestamp indicating a time when each blood glucose measurement value was obtained in the sequence of blood glucose measurement values.

5. The non-transitory computer readable medium of any preceding claim, wherein calculating the expected liquid drug delivery amount accounts for insulin onboard and peak insulin action.

6. The non-transitory computer readable medium of any preceding claim, wherein each of the plurality of advisory algorithms utilizes a different set of input parameters categorized as an aggressive input set, a standard input set, or a conservative input set for execution.

7. The non-transitory computer readable medium of any preceding claim, wherein the processor is operable to:
calculate the deviation between the expected liquid drug delivery amount and each respective advisory liquid drug delivery output generated by a plurality of advisory mode algorithms,
identify the advisory mode algorithm output with the minimum deviation from the expected liquid drug delivery amount,
and select the parameter settings of the advisory mode algorithm which generated the advisory liquid drug delivery output with the minimum deviation from the expected liquid drug delivery amount.

8. The non-transitory computer readable medium of any preceding claim, wherein the processor is operable to generate a recommendation based on the selected respective advisory liquid drug delivery amount.

9. The non-transitory computer readable medium of claim 1, wherein calculating the amount of the liquid drug expected to have been delivered by the drug delivery device, comprises the steps of:
calculating a user's average predicted glucose deviations in the next several cycles;
dividing the user's average predicted glucose deviations in the next several cycles by a TDI-based correction factor, wherein the quotient is the estimated correction insulin needed for the user;
determining a difference between an existing insulin on board from the estimated correction insulin needed for the user; and
dividing the difference by a length of insulin delivery history being investigated, which is scaled by an insulin peak time value, wherein the quotient of the division is additional insulin delivery needs for the user.

10. The non-transitory computer readable medium of claim 9, wherein calculating the amount of the liquid drug expected to have been delivered by the drug delivery device, further comprises the steps of:
determining a sum of all insulin delivered over a period of insulin delivery time; and
adding the sum of all the insulin delivered over the period of insulin delivery time to the additional insulin delivery needs for the user, wherein the result of the adding is the amount of the liquid drug expected to have been delivered by the drug delivery device.

11. The non-transitory computer readable medium of claim 1, wherein a first respective advisory algorithm of the plurality of advisory algorithms includes aggressive parameter settings, a second respective advisory algorithm of the plurality of advisory algorithms includes standard parameter settings, and a third respective advisory algorithm of the plurality of advisory algorithms includes conservative parameter settings.

12. A controller for an insulin delivery system, the controller comprising:
a processor; and
a memory storing instructions which, when executed by the processor, cause the processor to:
calculate, by a processor of a drug delivery device, an amount of a liquid drug that should have been delivered by the drug delivery device during a previous delivery cycle;
executing a plurality of advisory algorithms, wherein each respective advisory algorithm of the plurality of advisory algorithms has different parameter settings and is operable to generate an estimated liquid drug amount expected to have been delivered during the previous delivery cycle;
compare the estimated liquid drug amount generated by each respective advisory algorithm to the determined amount of the liquid drug that should have been delivered;
select the respective advisory algorithm that a result of the comparison indicates generated the estimated liquid drug amount that most closely matched the determined amount of the liquid drug that should have been delivered during the previous delivery cycle; and
use parameter settings of the selected respective advisory algorithm in the calculation of a next amount of the liquid drug to be delivered by the drug delivery device during a next delivery cycle.

13. The controller of claim 12, wherein calculating the amount of the liquid drug expected to have been delivered by the drug delivery device, comprises the steps of:
calculating a user's average predicted glucose deviations in the next several cycles;
dividing the user's average predicted glucose deviations in the next several cycles by a TDI-based correction factor, wherein the quotient is the estimated correction insulin needed for the user;
determining a difference between an existing insulin on board from the estimated correction insulin needed for the user; and
dividing the difference by a length of insulin delivery history being investigated, which is scaled by an insulin peak time value, wherein the quotient of the division is additional insulin delivery needs for the user.

14. The controller of claim 13, wherein calculating the amount of the liquid drug expected to have been delivered by the drug delivery device, further comprises the steps of:
determining a sum of all insulin delivered over a period of insulin delivery time; and
adding the sum of all the insulin delivered over the period of insulin delivery time to the additional insulin delivery needs for the user, wherein the result of the adding is the amount of the liquid drug expected to have been delivered by the drug delivery device.

15. The controller of claim 12, wherein a first respective advisory algorithm of the plurality of advisory algorithms includes aggressive parameter settings, a second respective advisory algorithm of the plurality of advisory algorithms includes standard parameter settings, and a third respective advisory algorithm of the plurality of advisory algorithms includes conservative parameter settings.
